# EUROPEAN PATENT SPECIFICATION

(11) **EP 0 911 315 B1**
(45) Date of publication and mention of the grant of the patent: **11.06.2003**
(21) Application number: 98811011.0
(22) Date of filing: 07.10.1998
(51) Int. Cl.: C07C 69/738, C07D 307/33, C07C 69/757, C07C 69/716, C07C 69/38, C07C 69/40, A61K 7/46, A01N 37/06, A01N 37/02

(54) **Beta-ketoester**
Beta-Ketoester
Bèta-cétoesters

(30) Priority: 21.10.1997 EP 97810783
(43) Date of publication of application: 28.04.1999
(60) Divisional application: 00126382.1
(73) Proprietor: Givaudan SA, 1214 Vernier-Genève (CH)
(72) Inventor: Anderson, Denise, Dr., 8032 Zürich (CH); Frater, Georg, Dr., 8400 Winterthur (CH)
(74) Representative: Patentanwälte Schaad, Balass, Menzl & Partner AG

(56) References cited:
- EP-A- 0 881 253
- EP-A- 0 891 962
- WO-A-98/07407
- FR-A- 1 319 516
- GB-A- 908 674
- US-A- 2 683 720
- ELISE BALAUX ET AL.: "Synthesis of Succinic Diesters via Reductive Coupling of alpha-Haloesters Using Samarium(II) Iodide and HMPA" TETRAHEDRON LETTERS., vol. 37, no. 6, 5 February 1996, pages 801-804, XP002091281 OXFORD GB
- WALTER KIMEL ET AL.: "Crotyl malonate,Methylvinylcarbinyl Malonate and Cinnamyl Cyanoacetate" JOURNAL OF THE AMERICAN CHEMICAL SOCIETY., vol. 66, no. 9, 8 September 1944, pages 1613-1614, XP002091282 DC US
- H.E.ZAUGG ET AL.: "Specific Solvent Effects in the Alkylation of Enolate Anions.III. Preparative Alkylations in Dimethylformamide" JOURNAL OF ORGANIC CHEMISTRY., vol. 26, no. 3, 31 March 1961, pages 644-651, XP002091283 EASTON US
- R.T.LALONDE ET AL.: "A Stereocontrolled Synthesis of (+-)-Anhydronupharamine.The 1H and 13C Nuclear Magnetic Resonance of Piperidine Nuphar Alkaloids" JOURNAL OF ORGANIC CHEMISTRY., vol. 42, no. 12, 10 June 1977, pages 2113-2118, XP002091284 EASTON US
- R.BRYAN MILLER ET AL.: "A Regiospecific Synthesis of alpha-Methylene Ketones" TETRAHEDRON LETTERS., no. 50, December 1973, pages 5037-5039, XP002091285 OXFORD GB
- CAREY: "Organic Chemistry, p. 789-793", 1992, MC GRAW HILL, NEW YORK
- MARCH: "Advanced Organic Chemistry, p. 378, 465 and 629", 1992, WILEY INTERSCIENCE, NEW YORK
- JUSTUS LIEBIGS ANNALEN DER CHEMIE 1977, pages 1557 - 1571
- TETRAHEDRON LETT. vol. 35, 1994, pages 2767 - 2770
- J. ORG. CHEM. vol. 52, 1987, pages 5425 - 5430
- J. CHEM. SOC. CHEM. COMMUN. 1989, pages 929 - 930
- INDIAN JOURNAL OF CHEMISTRY vol. 20A, 1981, pages 560 - 563
- TETRAHEDRON LETT. vol. 34, 1993, pages 6575 - 6578
- CHEM. PHARM. BULL vol. 37, 1989, pages 665 - 669
- JUSTUS LIEBIGS ANNALEN DER CHEMIE vol. 699, 1966, pages 195 - 205
- J. CHEM. SOC. PERKIN TRANS I 1991, pages 1667 - 1671
- J. AM. CHEM. SOC. vol. 113, 1991, pages 5467 - 5468
- TETRAHEDRON LETT. vol. 36, 1995, pages 4673 - 4676

## Description

The present invention relates to the use of beta-keto esters which are useful as precursors for organoleptic compounds, especially for flavours, fragrances and masking agents and antimicrobial compounds.

A principal strategy currently employed in imparting odours to consumer products is the admixing of the fragrance directly into the product. There are, however, several drawbacks to this strategy. The fragrance material can be too volatile and or too soluble, resulting in fragrance loss during manufacturing, storage, and use. Many fragrance materials are also unstable over time. This again results in loss during storage.

In many consumer products it is desirable for the fragrance to be released slowly over time. Micro-encapsulation and inclusion complexes with cyclodextrins have been used to help decrease volatility, improve stability and provide slow-release properties. However, these methods are for a number of reasons often not successful. In addition, cyclodextrins can be too expensive.

Fragrance precursors for scenting fabrics being washed in the presence of a lipase-containing detergents are described in WO 95/04809. The fragrance precursors contained in the detergent and/or in the softener are cleaved by the lipase and a single odoriferous compound, either an odoriferous alcohol or aldehyde or ketone is yielded. Thereby a prolonged scenting effect on the fabric is obtained.

Beta-amino ester compounds of perfume alcohols and their use as precursors for active alcohols which are released under alkaline conditions are described in the EP-A 0 771 786.
Beta-ketoester pro-accords for personal care and personal hygiene articles are disclosed in WO 98/07407. The fragrance compounds released by the pro-accords are alcohols only.

One object of the present invention is to provide new precursors for compounds with different activities. A further object of the invention is to provide new compounds which are stable under transport and storage conditions. A further object of the present invention is to provide precursor molecules supplying different active compounds simultaneously or successively.

The present invention relates to the use of beta-keto esters of the formula I in consumer products or in compositions for consumer products 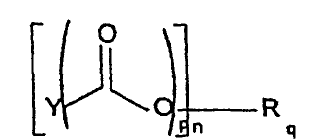 as precursors for organoleptic and antimicrobial compounds
wherein
Y is the residue of an organoleptic lactone of the formula YH,
R is the residue of a phenol or of a mono- or polyalcohol of the formula R-(OH)ₛ with s ≥ 1,
n = 1 or 2
p = q and p is 1 or 2,
whereby if n > 1 the rests Y may be different or the same.

The compounds of formula I are not limited to any particular stereoisomers, all possible stereoisomers (E/Z isomers, enantiomers, diastereomers) and all mixtures are thus included within the scope of the invention.

The compounds of formula I are mostly or nearly odourless at room temperature, atmospheric conditions and about 20 to 100 % relative humidity. However, under activating conditions, they are cleaved and one or more active compounds with organoleptic and/or antimicrobial properties are generated.

The activating conditions which lead to cleavage and the desired active compounds comprise the presence of skin bacteria, especially axilla bacteria, of an enzyme such as protease or lipase, elevated temperature or acidic or alkaline pH-values. Under activating conditions the beta-keto esters of formula I are cleaved into an unstable beta-keto acid and an alcohol or phenol or, then the beta-keto acid decomposes to an organoleptic lactone according to the following: 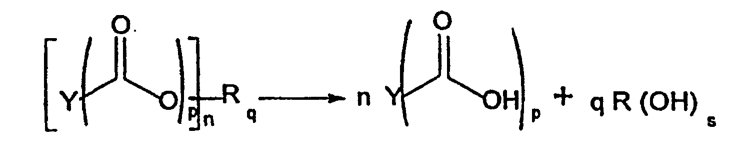 (thereby ROH may be an organoleptic alcohol or phenol)

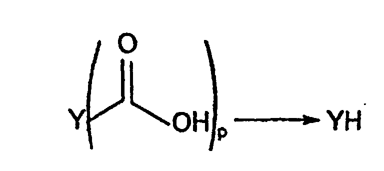

The compounds of formula I, upon cleavage, provide ketones or lactones and optionally alcohols or phenols having organoleptic and/or antimicrobial activity and therefore permit the development of useful consumer products with enhanced organoleptic and/or antimicrobial properties. The organoleptic lactones obtained are useful as fragrances, flavours and masking agents and antimicrobial agents. Therefore, the invention also relates to the use of all compounds of formula I as precursors for organoleptic compounds, e.g. flavours, fragrances, masking agents and as precursors for antimicrobial agents.

The beta-keto esters of formula I can act as fragrance precursors in personal care products, in laundry products, cleaning compositions, pet care products and environment scents such as air fresheners. They can also act as precursors for odour masking agents in the same products as the fragrance precursors. They also can act as precursors for antimicrobial agents. Further, they can act as flavour precursors in food and beverage products. The fragrance precursors and the precursors for odour masking agents as well as the flavour precursors of the invention may be used individually in an amount effective to enhance or to mask the characteristic odour or flavour of a material. More commonly, however, the compounds are mixed with other fragrance or flavour components in an amount sufficient to provide the desired odour or flavour characteristics.

Due to the in situ generation of the active compounds the desired effect is prolonged and the substantivity on different substrates is enhanced. If two or more active compounds are provided, they can be generated, depending on the precursor and/or the activating conditions, simultaneously or successively. Further, the precursors of the invention provide slow release of the active compounds.

Compounds of formula I wherein Y is the residue of a lactone are preferred.

Compounds of formula I wherein R = or the residue of a nonfragrant phenol or of a nonfragrant mono- or polyalcohol, especially having more than 3-C atoms are also preferred.

Compounds of formula I may generate the following lactones of formula HY
6-methyl-pyran-2-one
5-heptyl-dihydro-furan-2-one*
5-pentyldihydro-2(3H)-furanone*
5-(3-hexenyl)dihydro-5-methyl-(Z)-2(3H)-furanone
5-hexyldihydro-5-methyl-2(3H)-furanone
5-hexyldihydro-2(3H)-furanone*
5-octyldihydro-2(3H)-furanone
8-(1-methylethyl)-1-oxaspiro[4.5]-decan-2-one*
8-methyl-1-oxaspiro[4.5]-decan-2-one
8-ethyl-1-oxaspiro[4.5]-decan-2-one
5-(1,5-dimethyl-4-hexanyl)dihydro-2(3H)-furanone
2-oxo-5-butyl-tetrahydrofuran*
4-methyl-5-pentyl-dihydro-2(3H)-furan-2-one
5-hexyldihydro-5-methyl-2(3H)-furanone
dihydro-5-methyl-5-vinyl-2(3H)-furanone
octahydro-2H-1-benzopyran-2-one
tetrahydro-6-pentyl-2H-pyran-2-one
tetrahydro-6-hexyl-2H-pyran-2-one
tetrahydro-6-heptyl-2H-pyran-2-one
tetrahydro-6-(3-pentenyl)-(E)-2H-pyran-2-one
tetrahydro-6-(2-pentenyl)-(Z)-2H-pyran-2-one
(E)-oxacycloheptadec-10-en-one**
oxacyclohexadecan-2-one**
dodeca-12-olide
where by * indicates the preferred lactones and ** indicate the more preferred lactones.

Examples of organoleptic monoalcohols and phenols constituting the residue R- in the compounds of formula I and generated upon cleavage are:
amyl alcohol
hexyl alcohol*
2-hexyl alcohol*
heptyl alcohol*
octyl alcohol*
nonyl alcohol*
decyl alcohol*
undecyl alcohol*
lauryl alcohol*
myristic alcohol
3-methyl-but-2-en-1-ol*
3-methyl-1-pentanol
cis-3-hexenol*
cis-4-hexenol*
3,5,5-trimethyl hexanol
3,4,5,6,6-pentamethylheptan-2-ol*
citronellol*
geraniol*
oct-1-en-3-ol
2,5,7-trimethyl octan-3-ol
2-cis-3,7-dimethyl-2,6-octadien-1-ol
6-ethyl-3-methyl-5-octen-1-ol*
3,7-dimethyl-oct-3,6-dienol*
3,7-dimethyloctanol*
7-methoxy-3,7-dimethyl-octan-2-ol*
cis-6-nonenol*
5-ethyl-2-nonanol
6,8-dimethyl-2-nonanol*
2,2,8-trimethyl-7(8)-nonene-3-ol
nona-2,6-dien-1-ol
4-methyl-3-decen-5-ol*
dec-9-en-1-ol
benzylalcohol
2-methyl undecanol
10-undecen-1-ol
1-phenyl ethanol*
2-phenyl ethanol*
2-methyl-3-phenyl-3-propenol
2-phenyl propanol*
3-phenyl propanol*
4-phenyl-2-butanol
2-methyl-5-phenyl pentanol*
2-methyl-4-phenyl-pentanol*
3-methyl-5-phenyl-pentanol*
2-(2-methylphenyl)-ethanol*
4-(1-methylethyl)benzene methanol
4-(4-hydroxyphenyl)butan-2-one*
2-phenoxy ethanol*
4-(1-methylethyl)-2-hydroxy-1-methyl benzene
2-methoxy-4-methyl phenol
4-methyl phenol
anisic alcohol*
p-tolyl alcohol*
cinnamic alcohol*
vanillin*
ethyl vanillin*
eugenol*
isoeugenol*
thymol
anethol*
decahydro 2-naphthalenol
borneol*
cedrenol*
farnesol*
fenchyl alcohol*
menthol*
3,7,11-trimethyl-2,6,10-dodecatrien-1-ol
alpha ionol*
tetrahydro ionol*
2-(1,1-dimethylethyl)cyclohexanol*
3-(1,1-dimethylethyl)cyclohexanol*
4-(1,1-dimethylethyl)cyclohexanol*
4-isopropyl cyclohexanol
6,6-dimethyl-bicyclo[3.3.1]hept-2-ene-2-ethanol
6,6-dimethyl-bicyclo[3.1.1]hept-2-ene-methanol*
p-menth-8-en-3-ol*
3,3,5-trimethyl cyclohexanol
2,4,6-trimethyl-3-cyclohexenyl-methanol*
4-(1-methylethyl)cyclohexyl-methanol*
4-(1,1-dimethylethyl)cyclohexanol
2-(1,1-dimethylethyl)cyclohexanol
2,2,6-trimethyl-alpha-propyl cyclohexane propanol*
5-(2,2,3-trimethyl-3-cyclopentenyl)-3-methylpentan-2-ol*
3-methyl-5-(2,2,3-trimethylcyclopent-3-enyl) pent-4-en-2-ol*
2-ethyl-4(2,2,3-trimethylcyclopent-3-en-1-yl)but-2-en-1-ol*
4-(5,5,6-trimethylbicyclo[2.2.1]hept-2-yl)-cyclohexanol*
2-(2-methylpropyl)-4-hydroxy-4-methyl-tetrahydropyran*
2-cyclohexyl propanol*
2-(1,1-dimethylethyl)-4-methyl cyclohexanol*
1-(2-tert-butyl-cyclohexyloxy)-2-butanol*
1-(4-isopropyl-cyclohexyl)-ethanol*
2,6-dimethyl-oct-7-en-2-ol**
2,6-dimethyl-heptan-2-ol**
3,7-dimethyl-octa-1,6-dien-3-ol**
whereby * indicates the preferred organoleptic alcohols and phenols and ** indicate the more preferred organoleptic alcohols and phenols.

Examples of preferred non-organoleptic alcohols and phenols constituting the residue R- in the compounds of formula I are:
serine
tyrosine
threonine
7-hydroxy-4-methyl coumarin
benzyldimethyl-2-hydroxyethylammonium chloride
[polyoxyethylene (4) lauryl ether]
[polyoxyethylene (2) cetyl ether]
[polyoxyethylene (2) stearyl ether]

Other preferred non-organoleptic alcohols and phenols are those with an affinity to fibres or those used in cosmetics and laundry formulations. A list of suitable cosmetic alcohols and phenols can be found in the Cosmetic Ingredient Handbook edited by Joanne M. Nikitakis. Suitable surfactant alcohols can be found e.g. in Surfactants Europe edited by Gordon L. Hollis. Examples of alcohols and phenols with special affinity to fibres are those that contain one or more quaternary amine groups or silicon atoms.

Examples of polyalcohols constituting the residue R- in the compounds of formula I are:
diols such as: diethylene glycol, propylene glycol, triethylene glycol, 4,4'-bicyclohexyldiol, N,N'-bis-(2-hydroxyethyl) ethylenediamine, 1,3-bis-(4-hydroxybutyl)-1,1,3,3-tetramethyl-disiloxane, 1,4-bis-(hydroxymethyl)-cyclohexane
triols such as: glycerol, cis,cis-1,3,5-cyclohexanetriol, triethanolamine
sugars such as: furanoside and pyranoside sugars such as glucose, fructose
polymers such as: hydroxyethylcellulose, hydroxypropylcellulose.

It is a matter of course, that it is not possible to give a complete list of the organoleptic and/or antimicrobial lactones, alcohols and phenols and non-organoleptic, especially nonfragrant alcohols, phenols and polymeric alcohols which are generated as a result of the desired cleavage of the beta-keto esters of formula I by skin bacteria, by enzymes, by elevated temperatures or by acidic and/or alkaline pH-values. The skilled person is, however, quite aware of those ketones, lactones, alcohols and phenols which provide the desired organoleptic, e.g. fragrance, flavour and odour masking and/or antimicrobial effects.

The compounds of formula I may preferably be used as sustained release odorants and flavours but also to mask or attenuate undesirable odours or to provide additional odours not initially present in consumer products, i.e. personal care products such as cosmetic products destined for application to human skin such as underarm deodorants or antiperspirants or other deodorants contacting the body, or in hand lotions, hair care products such as shampoos and conditioners, baby powders, baby lotions, ointments, foot products, facial cleansers, body wipes, facial makeup, colognes, after-shave lotions, shaving creams, etc. Additional applications include laundry detergents, fabric softeners, fabric softener sheets, (automatic) dishwasher detergents and all purpose cleaners. Further applications are air fresheners and odorants, odour masking agents and/or antimicrobial agents.
The compounds I are also useful in the flavouring and aromatizing of cooked foods. Addition of the beta-keto esters either singly or as a mixture to a cake batter, e.g. a microwave cake batter, serves to impart appropriate baking aromas to the cake as it is heated in the microwave as well as impart flavouring in the finished product. Compounds I are also useful in the flavouring and aromatizing of beverages, e.g. hot beverages such as teas and instant beverages prepared by adding hot water to a powder. Compounds I can also act as slow release agents in acidic or alkaline beverages. Further, these compounds are also useful for flavouring and aromatizing tobacco products, e.g. cigarettes.

The amount required to produce the desired, overall effect varies depending upon the particular compounds of formula I chosen, the product in which it will be used, and the particular effect desired.

For example, depending upon the selection and concentration of the compound chosen, when a compound of the formula I is added either singly or as a mixture, e.g. to a deodorant or laundry product composition at levels ranging from 0.1 to 10 % by weight, or most preferred 0.25 to 4 % by weight, an odorant, i.e. at least one odoriferous ketone, lactone, alcohol or phenol in an "organoleptically effective amount" is released when the product is used. This newly formed odorant serves to enhance the odour of the product itself or of a fragrance present in the product.

Depending upon the selection and concentration, addition of the compounds I, either singly or as a mixture, to cigarette tobacco at levels ranging from 5 ppm to 50'000 ppm tends to enhance the smoking flavour and/or mask undesirable smoking odours. An important property of these compounds I is that the flavourant or odorant is covalently bound as a non-volatile compound and the flavourant or odorant is released only when the tobacco product is ignited and burns.

Addition of the compounds of formula I either separately or as a mixture at levels suitably ranging from 5 ppm to 50'000 ppm by weight onto the media enclosing the tobacco serves to incorporate the odorant/flavourant in the side-stream smoke of the tobacco. Air borne flavourants and/or odorants are thus introduced. This newly formed odorant or flavourant serves to enhance or mask the smoking odours depending upon selection and use levels of the compounds I.

As is evident from the above compilation of lactones, alcohols and phenols, a broad range of known odorants or flavours or mixtures can be generated from precursors of the invention. While manufacturing compositions the precursors of the invention may be used according to methods known to the perfumer, such as e.g. from W.A. Poucher, Perfumes, Cosmetics, Soaps, 2, 7th Edition, Chapman and Hall, London 1974.

The compounds of formula I can be prepared by using standard methods known to the skilled chemist. A wide variety of methods for their preparation is known. One example for this knowledge is Chem.Rev. (1995), 1065-1114.

Convenient methods are outlined in the Examples without limiting the invention thereto.

### Example 1

### 5-Heptyl-2-oxo-tetrahydro-furan-3-carboxylic acid phenethyl ester

A mixture of 24.2 g 5-heptyl-2-oxo-tetrahydro-furan-3-carboxylic acid methyl ester, 24.4 g phenylethyl alcohol and 1.4 g tetraisopropyl-ortho-titanate was heated under nitrogen atmosphere to 130°C for 5 minutes while methanol was distilled off. Then the reaction mixture was cooled to room temperature, poured into water and extracted with hexane. The organic layer was dried and evaporated to dryness. The residue was chromatographed to yield 23.9 g of a slightly yellow oil.
- NMR (CDCl3): 7.4-7.15 (m, 5H); 4.5-4.3 (m, 2H); 3.62-3.5 (m, 1H); 3.08-2.91 (m, 2H); 2.65-2.4 (m, 1H); 2.34-1,96 (m, 1H); 1.88-1.11 (m, 13H); 0.9 (t, 3H)

### Example 2

### 5-Heptyl-2-oxo-tetrahydro-furan-3-carboxylic acid 3,7-dimethyl-oct-6-enyl ester

According to the procedure of Example 1, 5-heptyl-2-oxo-tetrahydro-furan-3-carboxylic acid 3,7-dimethyl-oct-6-enyl ester was prepared from 5-heptyl-2-oxo-tetrahydro-furan-3-carboxylic acid methyl ester, 3,7-dimethyl-oct-6-en-1-ol and tetraisopropyl-ortho-titanate.

### Example 3

### 5-Heptyl-2-oxo-tetrahydro-furan-3-carboxylic acid 3,7-dimethyl-octa-2,6-dienyl ester

According to the procedure of Example 1, 5-heptyl-2-oxo-tetrahydro-furan-3-carboxylic acid 3,7-dimethyl-octa-2,6-dienyl ester was prepared from 5-heptyl-2-oxo-tetrahydro-furan-3-carboxylic acid methyl ester, 3,7-dimethyl-octa-2,6-dien-1-ol and tetraisopropyl-ortho-titanate.

### Example 4

### 5-Heptyl-2-oxo-tetrahydro-furan-3-carboxylic acid 2-phenoxy-ethyl ester

According to the procedure of Example 1, 5-heptyl-2-oxo-tetrahydro-furan-3-carboxylic acid 2-phenoxy-ethyl ester was prepared from 5-heptyl-2-oxo-tetrahydro-furan-3-carboxylic acid methyl ester, 2-phenoxyethanol, and tetraisopropyl-ortho-titanate.

### Example 5

### 3-Oxo-5-(2,6,6-trimethyl-cyclohex-2-enyl)-pentanoic acid 2-[3-oxo-5-(2,6,6-trimethyl-cyclohex-2-enyl)-pentanoyloxy]-ethyl ester

A mixture of 25.2 g 3-oxo-5-(2,6,6-trimethyl-cyclohex-2-enyl)-pentanoic acid methyl ester and 4.3 g ethylene glycol was slowly heated to 180°C while distilling off methanol. On completion, the reaction mixture was chromatographed over silica gel to yield a colourless liquid.
- NMR (CDCl₃): 5.35 (s, 2H); 4.37 (s, 4H); 3.48 (s, 4H); 2.55 (t, 4H); 2.05-1.86 (m, 4H);

### Example 6

### 5-Heptyl-2-oxo-tetrahydro-furan-3-carboxylic acid 2-(5-heptyl-2-oxo-tetrahydro-furan-3-ylcarbonyloxy)-ethyl ester

According to the procedure of Example 5, 5-heptyl-2-oxo-tetrahydro-furan-3-carboxylic acid 2-(5-heptyl-2-oxo-tetrahydro-furan-3-ylcarbonyloxy)-ethyl ester was prepared from 5-heptyl-2-oxo-tetrahydro-furan-3-carboxylic acid methyl ester and ethylene glycol.

### Example 7

Test cloth was washed with a lipase-containing detergent to which one or more of the precursors of Examples 1-6, had been added. Headspace analysis of the wet and dry laundry indicated the presence of the fragrances. The fragrance level was higher than when the test cloth was washed with a lipase-containing detergent to which one or more fragrances were added.

### Example 7

Test cloth was washed with a lipase-containing detergent and then a fabric softener, containing one or more of the precursors of Examples 1-6 was added to the rinse cycle. Headspace analysis of the wet and dry laundry indicated the presence of the fragrances. The fragrance level was higher than when the test cloth was washed with a lipase-containing detergent and then a fabric softener, containing one or more fragrances, was added to the rinse cycle.

### Example 8

The following set forth examples for the use of the compounds of the present invention in various products. The methods of forming the following compositions are well known to those skilled in the art. All formulations may contain additional ingredients known to those skilled in the art, e.g. colorants, opacifiers, buffers, antioxidants, vitamins, emulsifiers, UV absorbers, silicones and the like. All products can also be buffered to the desired pH. All values are % w/w. Delayed Release Fragrances stands in the following for compounds of Examples 1-6.

### a) Deo-colognes

| | | | | |
|---|---|---|---|---|
| Delayed Release Fragrances | 0.5 | 1.5 | 2.5 | 6.0 |
| Fragrance | 0.5 | 1.5 | 2.5 | 6.0 |
| Triclosan (Ciba Geigy) | 1.0 | - | 0.75 | 1.0 |
| Alcohol to | 100 | 100 | 100 | 100 |

### b) Deo-Sticks

| Antiperspirant | |
|---|---|
| Ethylene Glycol Monostearate | 7.0 |
| Shea butter | 3.0 |
| Neobee 1053 (PVO International) | 12.0 |
| Generol 122 (Henkel) | 5.0 |
| Kesscowax B (Akzo) | 17.0 |
| Dimethicone Dow Corning 345 | 35.0 |
| Aluminum Sesquichlorhydrate | 20.0 |
| Delayed Release Fragrances | 0.5 |
| Fragrance | 0.5 |
| | |

| Antiperspirant | |
|---|---|
| Steary Alcohol | 17.0 |
| Castor Wax | 3.0 |
| Talc | 5.0 |
| Aluminum Zirconium Tetrachlorhydrate | 20.0 |
| Delayed Release Fragrances | 1.0 |
| Fragrance | 1.0 |
| Dimethicone Dow 245 | to 100.0 |
| | |

| Clear Deodorant Stick | |
|---|---|
| Witconol APM | 43.0 |
| Propylene Glycol | 20.0 |
| Alcohol 39C | 20.0 |
| Demin Water | 7.0 |
| Monamid 150ADD | 5.0 |
| Millithix 925 | 2.0 |
| Ottasept Extra | 0.5 |
| Delayed Release Fragrances | 0.75 |
| Fragrance | 0.75 |
| | |

| Deodorant Stick | |
|---|---|
| Propylene Glycol | 69.0 |
| Demin Water | 21.8 |
| Triclosan | 0.2 |
| Sodium Stearate | 8.0 |
| Delayed Release Fragrances | 0.5 |
| Fragrance | 0.5 |
| | |

| Alcohol free Deodorant Stick | |
|---|---|
| PPG-3 Myristyl Ether (Witconol APM) | 36.0 |
| Propylene Glycol | 36.0 |
| Demin Water | 19.0 |
| Triclosan | 0.25 |
| Sodium Stearate | 7.75 |
| Delayed Release Fragrances | 0.5 |
| Fragrance | 0.5 |
| | |

| Antiperspirant Aerosol | |
|---|---|
| Absolute Ethanol | 15.0 |
| Zirconium Aluminum Tetrachlorhydrate | 5.0 |
| Bentone 38 | 1.5 |
| Delayed Release Fragrances | 0.75 |
| Fragrance | 0.75 |
| S-31 Hydrocarbon propellant | to 100.0 |
| | |

| Antiperspirant Pump | |
|---|---|
| Demin Water | 57.5 |
| Aluminum Sesquichlorhydrate | 20.0 |
| Triton X-102 (Union Carbide) | 2.0 |
| Dimethyl Isosorbide (ICI) | 20.0 |
| Delayed Release Fragrances | 0.25 |
| Fragrance | 0.25 |
| | |

| Roll-On | |
|---|---|
| Dimethicone DC 354 (Dow Corning) | 69.0 |
| Bentone 38 | 10.0 |
| Rezal 36 GP (Reheis Chem. Co.) | 20.0 |
| Delayed Release Fragrances | 0.5 |
| Fragrance | 0.5 |

### Example 58

### a) Fabric softener of the ester quat type (4 x concentrate):

| INGREDIENTS | CHEMICAL NAME | % |
|---|---|---|
| PHASE A | | |
| DEIONISED WATER | | to 100.0 |
| MgCl ₂ (saturated sol.) | Magnesium chloride | 1.0 |

| PHASE B | | |
|---|---|---|
| REWOQUAT WE 18 | di-(tallow carboxyethyl)hydroxy ethyl methylammonium methosulfate | 15.0 |
| GENAPOL O 100 | Ethoxylated fatty alcohol C16-C18 10EO | 2.0 |
| ANTIFOAM DB 31 | | 0.5 |

| PHASE C | | |
|---|---|---|
| ISOPROPYL ALCOHOL | | 3.0 |
| PRESERVATIVE | | Qs |
| PERFUME | | Qs |

### PROCESS:

While stirring and heating to 65° C, mix part A, then part B preheated to 65° C. After cooling to room temperature, add part C. The pH value of the finished product is 2.60. Recommended level of perfume is 1.0 %. Delayed release fragrances of Examples 1-39, 45-52 may be any part of this 1.0 %.

### b) Fabric softener of the ester quat type (1 x concentrate):

| INGREDIENTS | CHEMICAL NAME | % |
|---|---|---|
| PHASE A | | |
| DEIONISED WATER | | to 100.0 |

| PHASE B | | |
|---|---|---|
| REWOQUAT WE 18 | Di-(tallow carboxyethyl)hydroxy ethyl methylammonium methosulfate | 6.0 |
| DOBANOL 25-9 | Ethoxylated fatty alcohol C12-C15 9EO | 0.50 |
| ANTIFOAM DB 31 | | 0.10 |

| PHASE C | | |
|---|---|---|
| MYACIDE BT 30 | 2-bromo-2-nitropropane 1,3 diol | 0.03 |
| PROXEL GXL | Benzisothiazolinone sodium salt | 0.02 |
| PERFUME | | Qs |

### PROCESS:

While stirring and heating to 65° C, mix part A, then part B preheated to 65° C. After cooling to room temperature, add part C. The pH value of the finished product is 3.50. Recommended level of perfume: 0.3 %. Delayed release fragrences of Examples 1-6, may be any part of this 0.3 %.

### Example 59

A 1% solution of one or more of the products of Examples 1-6 in ethanol was applied to cigarette papers to produce levels of 5-50'000 ppm of each flavourant. The paper was incorporated in cigarettes and, upon burning, released a fragrant odor.

In the above examples, the following components were used:
- Triclosan: 5-chloro-2-(2,4-dichlorophenoxy)phenol
- Neobee 1053: glycerol tricaprate/caprylate
- Generol 122: soya sterol
- Kesscowax B: cetyl alcohol and glycol polymer
- Witconol APM: polypropylene glycol-3 myristyl ether
- Monamid 150 ADD: cocoamide diethanolamine
- Millithix 925: dibenzylidene sorbitol
- Ottasept Extra: quaternium 18 hectorite
- Bentone 38: quaternium 18 hectorite
- Triton X-102: octoxynol-13
- Dimethicone DC 354: mixture of fully methylated linear siloxanepolymers endblocked with trimethylsiloxy units
- Rezal 36 GP: Aluminum zirconium tetrachlorohydrexglycine

## Claims

1. Use of beta-keto esters of the formula I in consumer products or in compositions for consumer products 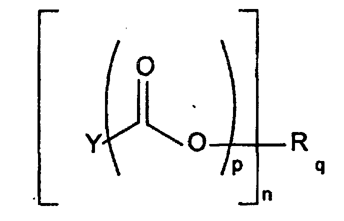 as precursors for organoleptic and antimicrobial compounds wherein
Y is the residue of a lactone of the formula YH,
R is the residue of a mono- or polyalcohol or phenol of the formula R-(OH)ₛ with s ≥ 1
n= 1 or 2
p = q, and p is 1 or 2,
whereby if n > 1 the residue Y may be different or the same.

2. Use according to claim 1 as fragrance precursors.

3. Use according to claim 1 as flavour precursors.

4. Use according to claim 1 as precursors for organoleptic masking agents.

5. Use according to claim 1 as precursors for antimicrobial agents.

6. Consumer product comprising the beta keto esters of formula I selected from the group consisting of underarm deodorants, antiperspirants, deodorants contacting the body, hand lotions, shampoos, conditioners, baby powders, baby lotions, ointments, foot products, facial cleansers, body wipes, facial makeup, colognes, after-shave lotions, shaving creams, laundry detergents, fabric softeners, fabric softener sheets, dishwasher detergents, automatic dishwasher detergents, all purpose cleaners, air fresheners, odorants, odour masking agents and/or antimicrobial agents, pet care products, environment scents, food and beverage products, tobacco products.

## Patentansprüche

1. Verwendung von Beta-Ketoestern der Formel I in Konsumprodukten oder in Zusammensetzungen für Konsumprodukte 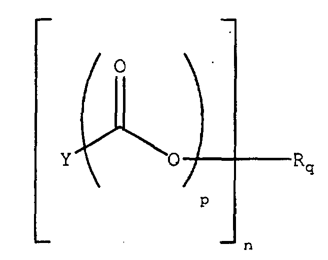 als Vorläufer für organoleptische und antimikrobielle Verbindungen, in der
Y der Rest eines Lactones der Formel YH ist,
R der Rest eines Mono- oder Poly-Alkohols oder Phenols der Formel R-(OH)ₛ mit s ≥ 1 ist,
n = 1 oder 2,
p = q, und p 1 oder 2 ist,
worin der Rest Y, wenn n > 1 ist, verschieden oder gleich sein kann.

2. Verwendung nach Anspruch 1 als Duftstoffvorläufer.

3. Verwendung nach Anspruch 1 als Geschmacksstoffvorläufer.

4. Verwendung nach Anspruch 1 als Vorläufer für organoleptische Maskierungsagentien.

5. Verwendung nach Anspruch 1 als Vorläufer für antimikrobielle Agentien.

6. Konsumprodukte, welche die Beta-Ketoester der Formel I enthalten, ausgewählt aus der Gruppe bestehend aus Unterarm-Deodorantien, Antiperspirantien, mit dem Körper in Kontakt kommende Deodorantien, Handlotionen, Shampoos, Haarspülungen, Babypudern, Babylotionen, Salben, Fussprodukten, Gesichtsreinigungsmitteln, Körperwischtüchern, Gesichts-Make-up, Rasierwassern, After-Shave-Lotionen, Rasiercremen, Wäschedetergentien, Textilweichmachern, Textilweichmacher-Blättern, Geschirrspül-Detergentien, Detergentien für automatische Geschirrspüler, Allzweckreinigern, Lufterfrischern, Geruchsstoffe, Geruchsmaskierungsagentien und/oder antimikrobiellen Agentien, Produkten zur Haustierpflege, Duftstoffen für die Umgebung, Nahrungs- und Getränkeprodukten, Tabakprodukten.

## Revendications

1. Utilisation de bêta-cétoesters de formule I dans des produits de consommation ou dans des compositions pour produits de consommation : 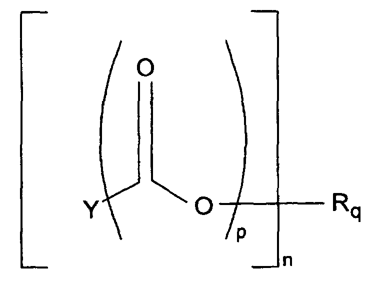 comme précurseurs de composés organoleptiques et antimicrobiens, dans laquelle :
Y est le résidu d'une lactone de formule YH,
R est le résidu d'un mono- ou d'un polyalcool ou de phénol de formule R-(OH)ₛ, s étant supérieur ou égal à 1,
n = 1 ou 2
p = q, et p vaut 1 ou 2,
moyennant quoi, si n est supérieur à 1, le résidu Y peut être identique ou différent.

2. Utilisation selon la revendication 1, en tant que précurseurs de parfums.

3. Utilisation selon la revendication 1, en tant que précurseurs d'arômes.

4. Utilisation selon la revendication 1, en tant que précurseurs d'agents masquants organoleptiques.

5. Utilisation selon la revendication 1, en tant que précurseurs d'agents antimicrobiens.

6. Produit de consommation comprenant les bêta-cétoesters de formule I choisis dans le groupe comprenant les déodorants pour les aisselles, les antitranspirants, les déodorants pour le corps, les lotions pour les mains, les shampooings, les démêlants, les poudres pour bébés, les lotions pour bébés, les pommades, les produits pour les pieds, les démaquillants pour le visage, les lingettes pour le corps, le maquillage pour le visage, les eaux de cologne, les lotions après-rasage, les crèmes à raser, les détergents pour le linge, les adoucissants pour textiles, les voiles adoucissants pour textiles, les détergents pour laver la vaisselle, les détergents pour lave-vaisselle automatique, les nettoyants multi-usages, les désodorisants, les agents odorisants, les agents masquant les odeurs et/ou les agents antimicrobiens, les produits de soins pour animaux, les senteurs naturelles, les produits alimentaires et les boissons, les produits à base de tabac.
